# EUROPEAN PATENT SPECIFICATION

(11) **EP 0 865 760 B1**
(45) Date of publication and mention of the grant of the patent: **12.01.2005**
(21) Application number: 97301925.0
(22) Date of filing: 21.03.1997
(51) Int. Cl.: A61B 5/00

(54) **Circumferential transillumination of anatomic junctions using light energy**
Umfangsdurchleuchtung von anatomischen Übergangen
Transillumination circonférentielle de jonctions anatomiques

(43) Date of publication of application: 23.09.1998
(73) Proprietor: Stryker Corporation, Santa Clara, California 95051 (US)
(72) Inventor: Fontenot, Mark G., Lafayette, Louisiana 70503 (US); Vining, James G., Yuma, Arizona 85364 (US); Feinberg, Richard, Bellingham, Washington 98225 (US)
(74) Representative: Findlay, Alice Rosemary

(56) References cited:
- US-A- 3 945 371
- US-A- 5 394 863
- US-A- 5 423 321
- US-A- 5 450 857

## Description

The present invention relates to methods and apparatus for circumferential transillumination of junctions between anatomic structures of a living body to allow surgeons to identify these junctions and guide incisions during surgical procedures and more specifically assisting in locating and/or avoiding such junctions.

Separating the cervix from the vagina during surgical procedures may be difficult. McCartney and Johnson (1995) reported on the use of a vaginal tube to separate the uterus from the vagina during laparoscopic hysterectomy which overcomes this difficulty by placing a wide bore plastic tube in the vagina to expose the cervico-vaginal junction and stretch the vaginal fornices. The stretched vagina falls away from the cervix as the surgeon attempts to locate the most distal aspect of the tube as it protrudes into the pelvic cavity. Palpation using an instrument during laparoscopy is frequently used to discover the distal aspect of the tube at which time the surgeon employs diathermy or other means to cut tissue overlying the tube. The protruding tube in the pelvic cavity presents a ridge which requires the surgeon to continually palpate the distal aspect of the tube in order to locate the cervico-vaginal junction and eventually to facilitate proper guidance of the incision. The procedure is difficult, time consuming and at times leads to problems.

US Patent No. 5394863 to Sanford discloses vaginal fomix illuminator. A fiber optic bundle that receives light from a light source is secured at a distal end to a proximal end of the illuminator. The illuminator comprises a pipe-like optical wave guide that carries light from its proximal end to its opposing distal end which is attached to an outwardly flared conical cup.

It is an object of the present invention to provide a device which circumferentially transilluminates a junction between anatomic structures using visible light or infrared light or combination of visible and infrared light so as to precisely locate such junction and warn the surgeon of imminent approach thereto.

The invention provides an instrument for illuminating an anatomical junction comprising a light source, a hollow member having a cylindrical region terminating at a distal end in a generally outwardly flared conical region, said conical region having a base with an end surface at its distal end, and a light emitting fiber having a proximal end connected to said light source and a distal end located in said conical region, characterised in that said light emitting fiber extends from the light source through said cylindrical region into said conical region such that light projects from the end surface of said base of said conical region to illuminate an anatomical junction.

The present invention overcomes and eliminates the need for the surgeon to palpate the distal aspect of a vaginal tube by using light energy to circumferentially transilluminate the cervico-vaginal junction. Furthermore, the invention can employ the same emission-detection technology described in U.S. Patent Number 5,423,321 entitled "Electronic Detection of Anatomic Passages Using An Infrared Emitting Catheter." The patent provides for transillumination of passages using an infrared emitting catheter. In one embodiment described in the patent, ureteral identification is intended to assist the surgeon with the management of the ureters during laparoscopic and open surgical procedures. When coupled to an infrared illuminator and placed in the ureter, an emitting fiber emits infrared light. Infrared transillumination of the ureter is not visible to the human eye. Thus electronic detection of the transilluminated ureter is facilitated using either a video system that employs an infrared sensitive imaging system or audible system which emits an audible tone when an infrared sensitive detector probe senses infrared light.

The differences between the aforementioned art and the instrument of the invention centers in at least one embodiment on circumferential transillumination of a junction compared to transillumination of a passage and the use of visible light with or without an infrared component. For example, the instrument may employ the same emitting fiber described in the patent. The emitting segment of the emitting fiber is circumferentially bonded to the distal aspect of a cup or tube seated substantially in contact with the cervico-vaginal junction thus resulting in circumferential junctional transilluminator of the invention. Therefore, transferring light energy to the emitting fiber and the emitting segment at the distal aspect of the circumferential junctional transilluminator permits detection of the transilluminated junction using the same method and devices described in the patent. Visible light, however, may also be used as another energy band to transilluminate the junction, in which case the eye or a typical medical endoscopic camera or the InfraVision camera could be used to detect the junction.

The instrument is described as employed to precisely locate the cervico-vaginal junction to reduce the danger to this junction particularly during performance of a hysterectomy. The instrument referred to as the CJT, and associated detection system is not limited to such use, but this use is illustrative of the utility of the instrument.

Visible light energy with or without a component of infrared light energy or infrared light energy with or without a component of visible light energy can be used to transilluminate the cervico-vaginal junction using the CJT. The infrared emission-detection technology of the '321 patent is currently in commercial use as the InfraVision System® with an application for ureteral identification and is readily applicable to the present invention. When the light energy emitter is coupled to the InfraVision IR Illuminator and placed in the ureter, the emitting fiber emits infrared light. Infrared transillumination of the ureter is not visible to the human eye. Detection of the transilluminated ureter is facilitated using either the InfraVision Imaging System or the Infravision Detector Probe.

To combine the instrument with the art described in the patent requires the CJT be coupled to the InfraVision IR Illuminator to provide modulated infrared light energy to transilluminate the cervico-vaginal junction. Since the human eye is not sensitive to infrared light energy, the InfraVision Imaging System and/or the InfraVision Detector Probe is used to locate the cervico-vaginal junction.

The InfraVision IR Illuminator consists of two electronic modules, a laser light source and light sensor housed in one unit. The InfraVision IR Illuminator is DC powered through an AC/DC power supply; is non-sterile; and reusable. The InfraVision IR Illuminator houses two variable 250 mW infrared laser diodes (laser light source); and has a photodetector and circuitry that is tuned to the modulation frequency and wavelength of the laser diodes (light sensor). The emitting fiber residing at the most distal aspect of the CJT is coupled to the InfraVision IR Illuminator which provides infrared light energy to transilluminate the junction. The InfraVision Imaging System consists of an infrared sensitive camera and camera control unit. The InfraVision Imaging System is sensitive to visible and infrared light, and is intended to detect the specific wavelength of the infrared transilluminated cervico-vaginal junction and display an image of the junction on a video monitor.

The CJT for the cervico-vaginal junction takes the form of a hollow cone with a narrow light emitting surface or edge at the base of the most distal aspect of the cone. The hollow cone is made preferably from plastic. Attached to the apex of the cone is a handle that facilitates introduction of the cone into the vaginal canal and which is used to press the base of the cone against the cervico-vaginal junction. The emitting fiber is housed in the handle and emerges at the apex of the cone and is secured to the edge of the base or most distal aspect of the cone. The light emitting segment diffuses light radially. The proximal end of the emitting fiber has an optical connector that couples to the infrared laser diodes of the InfraVision IR Illuminator. When the CJT is coupled to the InfraVision IR Illuminator and its distal end is placed against the cervico-vaginal junction, the CJT is transilluminated into the pelvic cavity.

For circumferential infrared transillumination of the cervico-vaginal junction, the CJT employed with the InfraVision is used as follows. After the vaginal area is prepared for surgery, the distal aspect of the CJT is introduced into the vaginal canal and placed over the cervix and against the cervico-vaginal junction. The emitting fiber is connected to the InfraVision IR Illuminator which launches infrared light energy into the emitting fiber. The cervico-vaginal junction is transilluminated since the emitting segment of the emitting fiber, which is bonded to the edge of the cone, allows light to diffuse radially and traverse into the abdomino-pelvic cavity. During laparoscopic procedures, the InfraVision Imaging System detects and projects the infrared transilluminated cervico-vaginal junction onto the video monitor. During difficult surgical cases when the cervico-vaginal junction is embedded in dense fibrous tissue, the Detector Probe can be introduced into the pelvic area through a trocar. Visualization of the Detector Probe tip on the video monitor allows the surgeon to position and maneuver the Detector Probe in the approximate area of the infrared transilluminated cervico-vaginal junction. The Detector Probe is positioned and manoeuvered until an audible sound is broadcast from the Light Sensor panel of the InfraVision IR Illuminator, which indicates detection of the infrared transilluminated cervico-vaginal junction. During open surgical procedures, the Detector Probe is introduced into the abdomen and pelvic areas and positioned in the approximate area of the cervico-vaginal junction. The Detector Probe is maneuvered until an audible sound is broadcast from the InfraVision IR Illuminator, which indicates detection of the infrared transilluminated cervico-vaginal junction.

The above and other features, objects and advantages of the present invention, together with the best means contemplated by the inventor thereof for carrying out the invention will become more apparent from reading the following description of a preferred embodiment and perusing the associated drawings in which:
Figure 1 is a longitudinal view of the CJT using an emitting fiber with the emitting segment bonded circumferentially to the distal edge of a cone;
Figure 2 is an isometric view of the CJT using an emitting fiber with the emitting segment bonded circumferentially to the distal edge of a cone;
Figure 3 is an end view of the distal edge of a cone showing the emitting fiber emerging from the handle and the emitting segment bonded to the edge;
Figure 4 shows a side view of the emitting fiber with the emitting segment used in the fabrication of the CJT;
Figure 5 is a longitudinal view of the CJT using two emitting fibers embedded into the handle and into the wall of a transparent cone;
Figure 6 is an isometric view of the CJT using two emitting fibers embedded into the handle and into the wall of a transparent cone;
Figure 7 shows a side of the emitting fiber without the emitting segment used in the fabrication of a transparent CJT;
Figure 8 is an end view of the distal aspect of a transparent cone with the emitting fiber embedded into the wall of a cone;
Figure 9a,9b and 9c are longitudinal views of the transparent cone showing the lateral, forward, and inward bevels resulting in light that is directed laterally, forward, or inwardly from the beveled edge of the transparent cone;
Figure 10a,10b and 10c show a side and cross sectional views of a CJT constructed from a bundle of emitting fibers;
Figure 11 shows a side view of a CJT constructed of transparent plastic coupled to a fiberoptic optic light guide for coupling to a light source;
Figure 12 illustrates the CJT placed at the cervico-vaginal junction and associated with the InfraVision IR Illuminator along with the InfraVision Imaging System during a surgical procedure.
Figure 13 illustrates the CJT connected to a visible light source and placed at the cervico-vaginal junction while using a typical endoscopic camera for detection of the transilluminated junction during a surgical procedure;
Figure 14 is an intra-pelvic view of the transilluminated cervico-vaginal junction as seen on the video monitor using the InfraVision Imaging System.

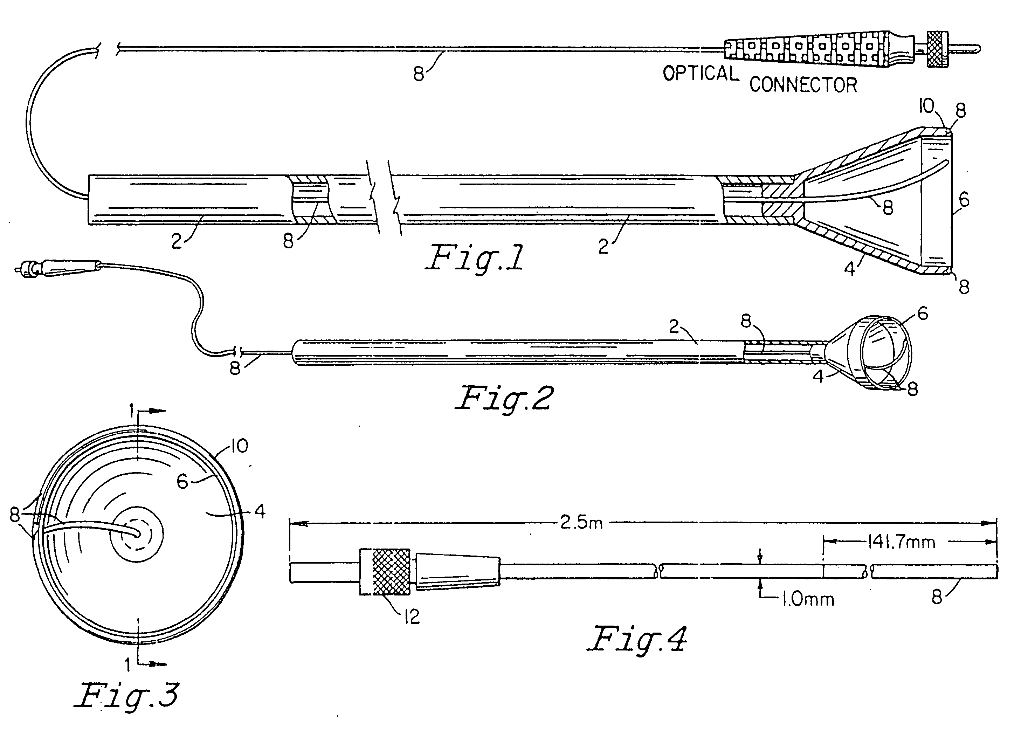

Referring to Figures 1, 2, 3 and 4 of the accompanying drawings, a CJT in accordance with the invention comprises a handle 2 attached to the virtual apex of a hollow cone 4. The distal aspect of the cone has an edge 6 to receive an emitting fiber 8. The distal portion of the emitting fiber 8 (ESKA SK-40) is scored at 10 to form the emitting segment as shown in Figure 4. The region 10 of the fiber which is circumferentially bonded to the edge 6 of the cone 4 using epoxy or the like. The emitting fiber has an optical connector 12 (SMA-905) fitted at its proximal end. The emitting fiber is secured to the inside wall 14 of the cone 4 as it passes out of the handle. The emitting segment bonded to the edge of the cone allows light to exit the wall of the end of the emitting fiber 8 resulting in a circumferential light emitting junctional transilluminator.

The emitting segment can be selectively abraded or scored so as to allow light to be emitted such that the entire cervico-vaginal junction (circumferential transillumination) or only a portion of the junction (semicircumferential) is transilluminated. The outer diameter of the cone 4 in a typical rendition of the invention shown in Figures 1, 2, and 4 is 45 mm with an edge thickness of 1mm. The length of the emitting segment circumferentially bonded to the edge is approximately 141.4 mm and the radius of the distal end of the cone 4 is approximately 22.5 mm. The length of the handle shown in Figures 2, and 3 is 45 cm and the length of the emitting fiber is 2.5 m.

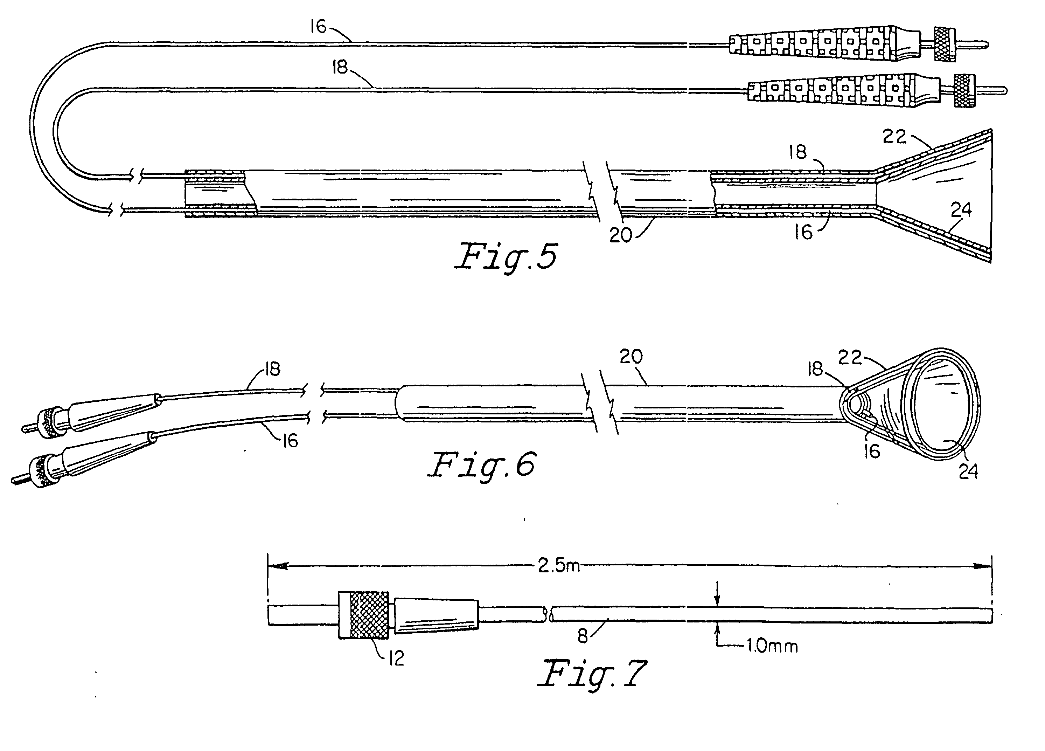

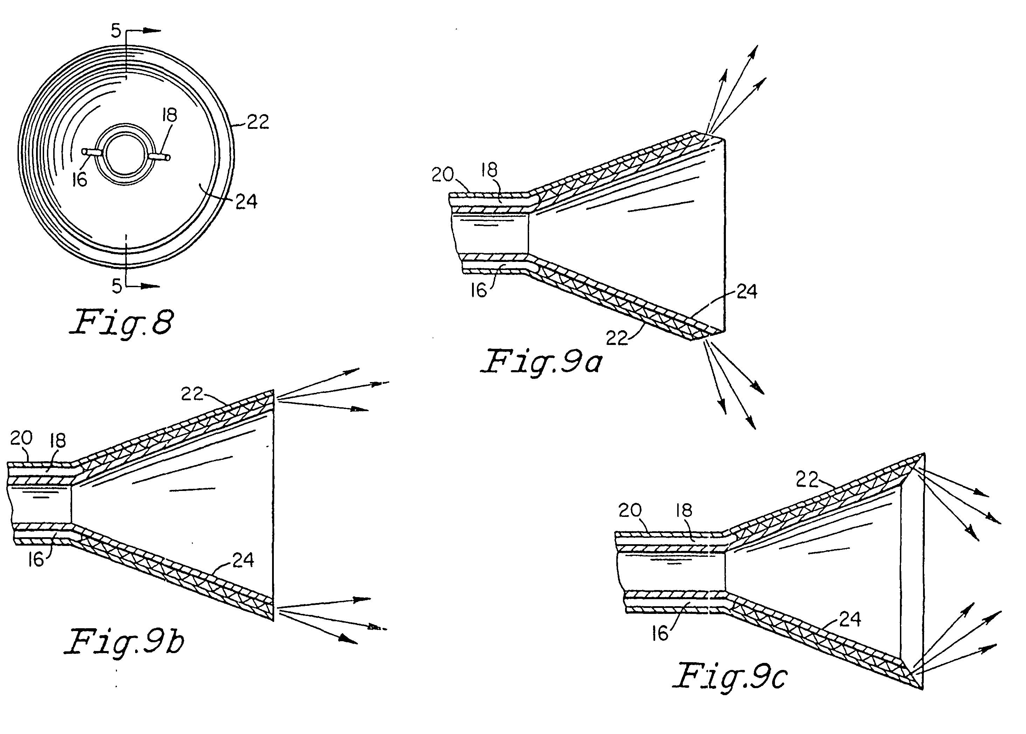

Referring now to Figures 5, 6, 7, and 8 of the drawings, the CJT is fabricated from a clear transparent polymer such as polymethylmethacrylate or an acrylic with two emitting fibers 16 and 18 embedded in wall 20 of a cone 22. The emitting fiber 16 shown in Figures 5, 6, and 7 is depicted in Figure 8 and has a flat polished end without the scored or abraded emitting segment. The end of the emitting fiber is embedded in the wall 20 of the cone such that light is directed parallel to the outer surface of the cone. The edge 24 of the cone can be beveled to direct light exiting the edge either laterally (Figure 9a), forward (Figure 9b), and/or inward (Figure 9c). The beveled edge 24 of the cone 20 also serves as a template or guide for the surgeon to make an incision along the light line, using the bevel as an incisional platen.

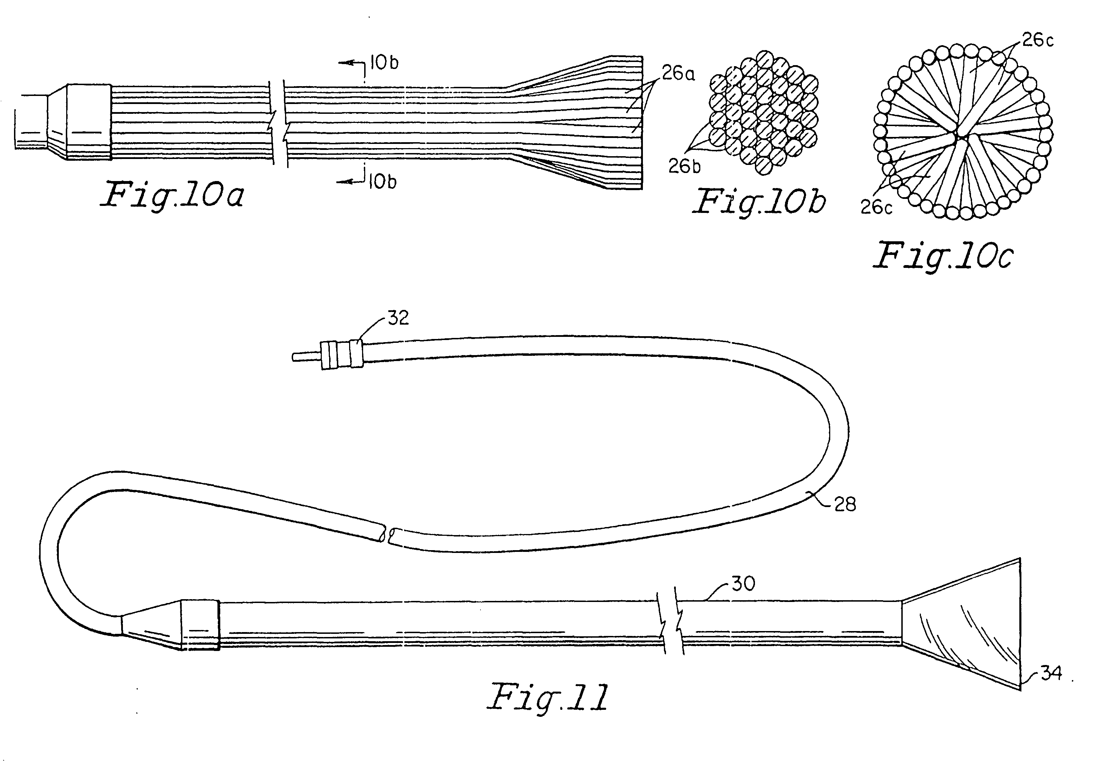

Efficiency can be improved significantly by increasing the number of emitting fibers embedded in the wall of the cone and/or having the end of the emitting fibers positioned closer to the edge. Figures 10a, 10b, and 10c are drawings showing a CJT constructed of many emitting fibers designated by the reference numerals 26a, 26b, and 26c respectively.

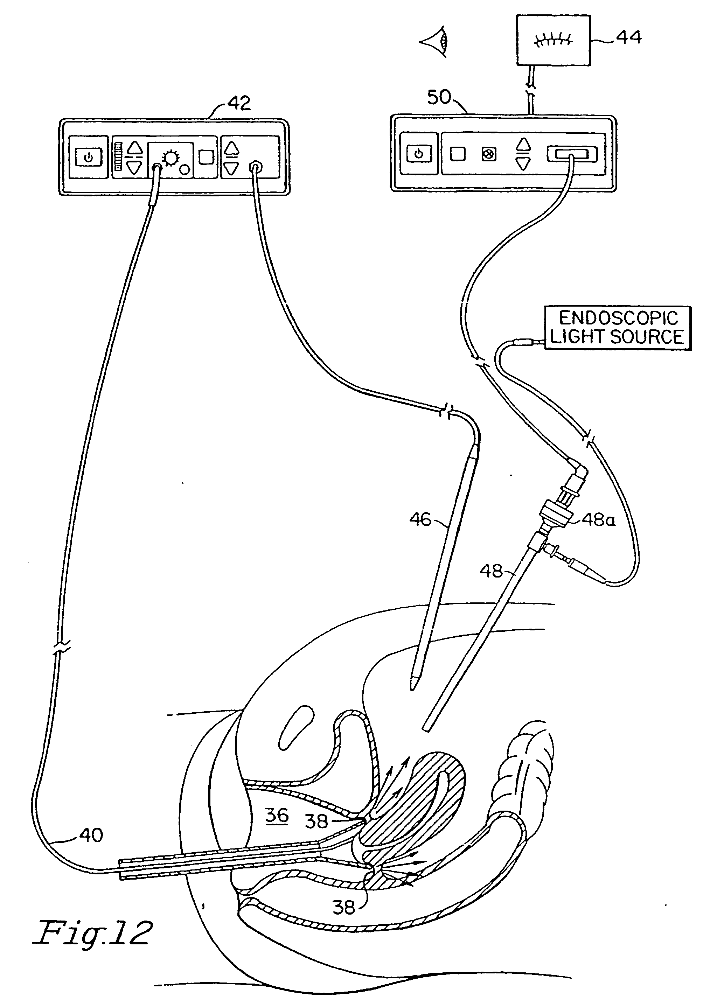

Referring to Figure 11 there is shown a CJT fabricated from cast polymethymethacrylate or an acrylic that has a fiberoptic bundle 28 attached to the proximal end of a handle 30. Optical connector 32 of the fiberoptic cable can be coupled to a high power light source such as a 300 watt xenon light source. When coupled to the light source, light traverses the entire length of the fiberoptic light cable and CJT and exits at beveled edge 34 of the cone

Referring to Figure 12 of the drawings, the CJT traverses the vaginal canal 36 and is positioned at the cervico-vaginal junction 38. An emitting fiber 40 is connected to the InfraVision IR Illuminator 42 as described in the '321 patent. The illuminator 42 launches light energy into the emitting fiber 40 and transilluminates the cervico-vaginal junction 38. A detector probe 46 may be used to locate the junction 38 by sensing, in this case, the infrared emissions through the junction and provides an audible visual indication when the probe approaches or is located at the junction. A camera 48a on endoscope 48 may provide light images via an imaging system 50 to the monitor 44, all as set forth in the aforesaid '321 patent and incorporated herein by reference.

Infrared laser diodes of the illuminator 42 can be modulated such that the infrared transilluminated junction appears on video monitor 44 to be "blinking" or the junction appears transilluminated continuously. In this figure, the CJT shown in Figures 1, 2, 9, and 10 may be employed as well as others disclosed herein. During laparoscopic procedures either by open abdominal surgery or by instruments inserted into the pelvic region through a trocar, the surgeon is able to detect the junction by either or both of the sensing means and can cut along the junction. The edge of the base of the cone when properly seated provides a guide for the scalpel. During difficult surgical cases when the cervico-vaginal junction is embedded in dense fibrous tissue, the combination of the detection probe and endoscope becomes quite useful. The Detector Probe can be introduced into the pelvic area through a trocar and visualization of the Detector Probe tip on the video monitor allows the surgeon to position and maneuver the Detector Probe in the approximate area of the infrared transilluminated cervico-vaginal junction. The Detector Probe is positioned and maneuvered until an audible sound is broadcast from the Light Sensor panel of the InfraVision IR Illuminator, which indicates detection of the infrared transilluminated cervico-vaginal junction. During open surgical procedures, the Detector Probe is introduced into the abdomen and pelvic areas and positioned in the approximate area of the cervico-vaginal junction. The Detector Probe is maneuvered until an audible sound is broadcast from the InfraVision IR Illuminator, which indicates detection of the infrared transilluminated cervico-vaginal junction thus permitting precise locating of the junction.

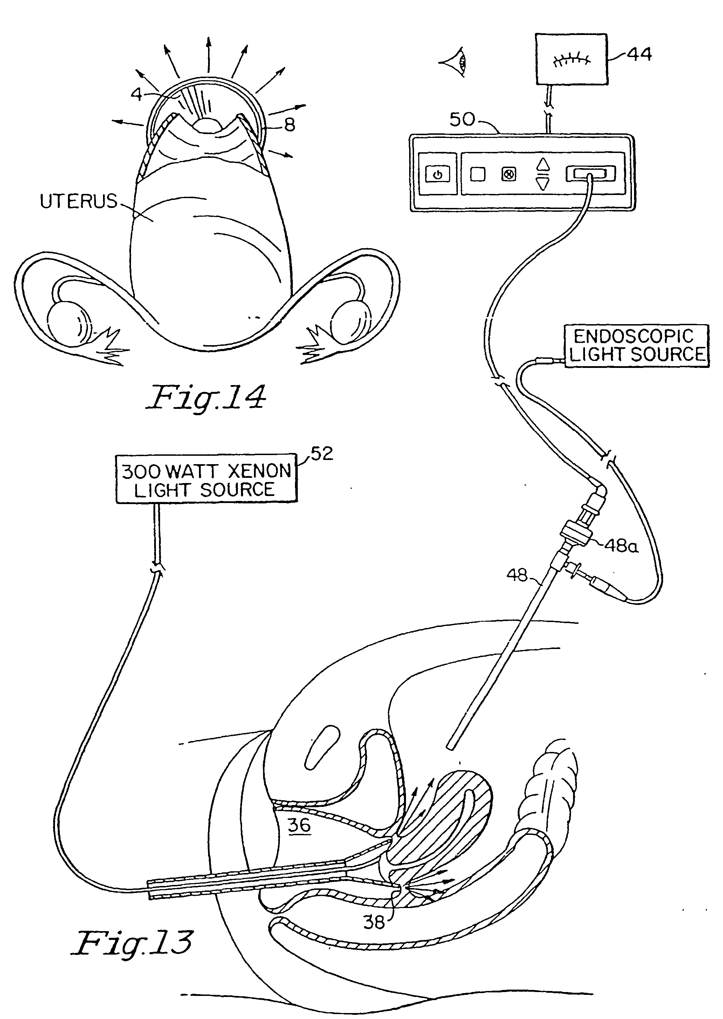

In Figure 13, the InfraVision IR Illuminator has been replaced with a 300 watt xenon light source 52. During endoscopic or open surgical procedures, the transilluminate cervico-vaginal junctional 38 can be viewed on a video monitor 44 by using an endoscopic camera 48a or can be seen with direct un-aided vision of the eye.

Referring to Figure 14, there is illustrated an intrapelvic view of the transilluminated cervico-vaginal junction using the present invention. Uterus 56 has been severed in this Figure and the base of the cone is visible.

## Claims

1. An instrument for illuminating an anatomical junction comprising a light source, a hollow member (2) having a cylindrical region terminating at a distal end in a generally outwardly flared conical region (4, 20), said conical region (4) having a base with an end surface (6, 24, 34) at its distal end, and a light emitting fiber (8, 16, 18, 28, 40) having a proximal end connected to said light source and a distal end (10) located in said conical region (4 20), said light emitting fiber (8, 16, 18, 28, 40) extending from the light source through said cylindrical region into said conical region such that light projects from the end surface (6, 24, 34) of said base of said conical region to illuminate an anatomical junction.

2. An instrument for illuminating an anatomical junction according to claim 1, wherein the distal end (10) of the light emitting fiber extends to the base of the conical region (4, 20).

3. An instrument for illuminating an anatomical junction according to claim 1 or claim 2, whereinthe distal end of said hollow member has a generatty flat end (6) in a plane perpendicular to an axis of said hollow member, and said light emitting fiber (8) is secured to the flat end of said hollow member.

4. An instrument for illuminating an anatomical junction according to any preceding claim, wherein said light source is an infrared source of light (42).

5. An instrument for illuminating an anatomical junction according to any preceding claim, further comprising at least one detector (46, 48, 48a) for detecting the light that projects from the end surface of said base.

6. An instrument for illuminating an anatomical junction according to claim 5 as dependent on claim 4, said detector comprises a detector (46, 48, 48a) of infrared energy.

7. An instrument for illuminating an anatomical junction according to claim 6, wherein said detector comprises a camera (48a).

8. An instrument for illuminating an anatomical junction according to claim 7, further comprising a monitor (44) for displaying images detected by said camera (48a).

9. An instrument for illuminating an anatomical junction according to claim 7, wherein said camera is a video camera, and said instrument includes a monitor (44) for displaying images produced by said video camera (48a).

## Patentansprüche

1. Instrument zum Ausleuchten einer anatomischen Verbindung, enthaltend eine Lichtquelle, ein hohles Element (2) mit einem zylindrischen Bereich, der an einem distalen Ende in einem im Wesentlichen nach außen geweiteten, konischen Bereich (4, 20) endet, wobei der konische Bereich (4) eine Basis mit einer Endoberfläche (6, 24, 34) an ihrem distalen Ende besitzt, und eine Licht emittierende Faser (8, 16, 18, 28, 40), die ein proximales Ende aufweist, welches mit der Lichtquelle verbunden ist, sowie ein distales Ende (10) besitzt, welches in dem konischen Abschnitt (4, 20) angeordnet ist, wobei die Licht abstrahlende Faser (8, 16, 18, 28, 40) sich von der Lichtquelle durch den zylindrischen Bereich in den konischen Bereich erstreckt, so dass Licht von der Endoberfläche (6, 24, 34) der Basis des konischen Bereichs abgestrahlt wird, um eine anatomische Verbindung zu befeuchten.

2. Instrument zur Beleuchtung einer anatomischen Verbindung gemäß Anspruch 1, wobei das distale Ende (10) der Litht abstrahlenden Faser sich zu der Basis des konischen Bereichs (4, 20) erstreckt.

3. Instrument zur Beleuchtung einer anatomischen Verbindung nach Anspruch 1 oder 2, wobei das distale Ende des hohlen Elements ein im Wesentlichen ebenes Ende (6) in einer Ebene senkrecht zu einer Achse des hohlen Elements besitzt und wobei die Licht abstrahlende Faser (8) an dem ebenen Ende des hohlen Elements angebracht ist.

4. Instrument zur Beleuchtung einer anatomischen Verbindung nach irgendeinem der vorstehenden Ansprüche, wobei die Lichtquelle eine Infrarotlichtquelle (42) ist

5. Instrument zur Beleuchtung einer anatomischen Verbindung nach irgendeinem der vorstehenden Ansprüche, weiterhin enthaltend zumindest einen Detektor (46, 48, 48a) zum Erfassen des Lichts, welches von der Endoberfläche der Basis abgestrahlt wird.

6. Instrument zur Beleuchtung einer anatomischen Verbindung nach Anspruch 5, soweit er von Anspruch 4 abhängig ist, wobei der Detektor einen Detektor (46, 48, 48a) für Infrarotenergie enthält

7. Instrument zur Beleuchtung einer anatomischen Verbindung nach Anspruch 6, wobei der Detektor eine Kamera (48a) enthält.

8. Instrument zur Beleuchtung einer anatomischen Verbindung nach Anspruch 7, weiterhin enthaltend einen Monitor (44) zum Anzeigen von Bildern, die durch die Kamera (48a) erfasst werden.

9. Instrument zur Beleuchtung einer anatomischen Verbindung nach Anspruch 7, wobei die Kamera eine Videokamera ist und das Instrument einen Monitor (44) zum Anzeigen von Bildem, welche durch die Videokamera (48a) erzeugt werden, enthält.

## Revendications

1. Instrument pour éclairer une jonction anatomique comprenant une source de lumière, un élément creux (2) ayant une région cylindrique se terminant au niveau d'une extrémité distale dans une région conique généralement évasée vers l'extérieur (4, 20), ladite région conique (4) ayant une base avec une surface d'extrémité (6, 24, 34) au niveau de son extrémité distale, et une fibre électroluminescente (8, 16, 18, 28, 40) ayant une extrémité proximale raccordée à ladite source de lumière et une extrémité distale (10) située dans ladite région conique (4, 20), ladite fibre électroluminescente (8, 16, 18, 28, 40) s'étendant de la source de lumière en passant par ladite région cylindrique dans ladite région conique de sorte que la lumière se projette depuis la surface d'extrémité (6, 24, 34) de ladite base de ladite région conique pour éclairer une jonction anatomique.

2. Instrument pour éclairer une jonction anatomique selon la revendication 1, dans lequel l'extrémité distale (10) de la fibre électroluminescente s'étend vers la base de la région conique (4, 20).

3. Instrument pour éclairer une jonction anatomique selon la revendication 1 ou la revendication 2, dans lequel l'extrémité distale dudit élément creux a une extrémité généralement plate (6) dans un plan perpendiculaire à un axe dudit élément creux, et ladite fibre électroluminescente (8) est fixée sur l'extrémité plate dudit élément creux.

4. Instrument pour éclairer une jonction anatomique selon l'une quelconque des revendications précédentes, dans lequel ladite source de lumière est une source de lumière infrarouge (42).

5. Instrument pour éclairer une jonction anatomique selon l'une quelconque des revendications précédentes, comprenant en outre au moins un détecteur (46, 48, 48a) pour détecter la lumière qui se projette à partir de la surface d'extrémité de ladite base.

6. Instrument pour éclairer une jonction anatomique selon la revendication 5 dépendant de la revendication 4, ledit détecteur comprend un détecteur (46, 48, 48a) d'énergie infrarouge.

7. Instrument pour éclairer une jonction anatomique selon la revendication 6, dans lequel ledit détecteur comprend une caméra (48a).

8. Instrument pour éclairer une jonction anatomique selon la revendication 7, comprenant en outre un écran (44) pour afficher les images détectées par ladite caméra (48a).

9. Instrument pour éclairer une jonction anatomique selon la revendication 7, dans lequel ladite caméra est une caméra vidéo, et ledit instrument comprend un écran (44) pour afficher les images produites par ladite caméra vidéo (48a).
